# EUROPÄISCHE PATENTSCHRIFT

(11) **EP 2 040 691 B1**
(45) Veröffentlichungstag und Bekanntmachung des Hinweises auf die Patenterteilung: **08.06.2011**
(21) Anmeldenummer: 07764851.7
(22) Anmeldetag: 26.06.2007
(51) Int. Cl.: A61K 31/197, A61K 47/18, A61P 9/00

(54) **WÄSSRIGE ARZNEIMITTELFORMULIERUNG VON 4-[((4-CARBOXYBUTYL)-{2-[(4-PHENETHYL-BENZYL)OXY]- PHENETHYL}AMINO)METHYL]BENZOESÄURE**
AQUEOUS PHARMACEUTICAL FORMULATION OF 4-[((4-CARBOXYBUTYL)-{2-[(4-PHENETHYL-BENZYL)OXY]-PHENETHYL}AMINO)METHYL]BENZOIC ACID
FORMULATION MÉDICAMENTEUSE AQUEUSE D'ACIDE 4-[((4-CARBOXYBUTYL)-{2-[(4-PHÉNÉTHYL-BENZYL)OXY]-PHÉNÉTHYL}AMINO)MÉTHYL]BENZOÏQUE

(30) Priorität: 06.07.2006 DE 102006031175
(43) Veröffentlichungstag der Anmeldung: 01.04.2009
(73) Patentinhaber: Bayer Schering Pharma Aktiengesellschaft, 13353 Berlin (DE)
(72) Erfinder: KÜHN, Bernd, 60323 Frankfurt (DE); WAGNER, Daniel, 65396 Walluf (DE)
(86) Internationale Anmeldenummer: PCT/EP2007/005625
(87) Internationale Veröffentlichungsnummer: WO 2008/003414

(56) Entgegenhaltungen:
- WO-A-2005/042022
- STASCH J-P ET AL: "NO- and haem-independent activation of soluble guanylyl cyclase: molecular basis and cardiovascular implications of a new pharmacological principle" BRITISH JOURNAL OF PHARMACOLOGY, BASINGSTOKE, HANTS, GB, Bd. 136, Nr. 5, 2002, Seiten 773-783, XP002403267 ISSN: 0007-1188
- BEHRENDS S: "DRUGS THAT ACTIVATE SPEICIFC NITRIC OXIDE SENSITIVE GUANYLYL CYCLASE ISOFORMS INDEPENDENT OF NITRIC OXIDE RELEASE" CURRENT MEDICINAL CHEMISTRY, BENTHAM SCIENCE PUBLISHERS BV, BE, Bd. 10, Nr. 4, Februar 2003 (2003-02), Seiten 291-301, XP009038710 ISSN: 0929-8673

## Beschreibung

Die vorliegende Erfindung betrifft eine wässrige Arzneimittelformulierung, die 4-[((4-Carboxybutyl)-{2-[(4-phenethylbenzyl)oxy]phenethyl}amino)methyl]benzoe-säure oder ein Salz davon und Amino-2(hydroxymethyl)-2-propandiol-1,3 (Trometamol) enthält.

4-[((4-Carboxybutyl)-{2-[(4-phenethylbenzyl)oxy]phenethyl}amino)methyl]benzoesäure (Verbindung 1) ist ein Aktivator der löslichen Guanylatcyclase mit Wirkung auf das Herz-Kreislaufsystem und entspricht der folgenden Formel:

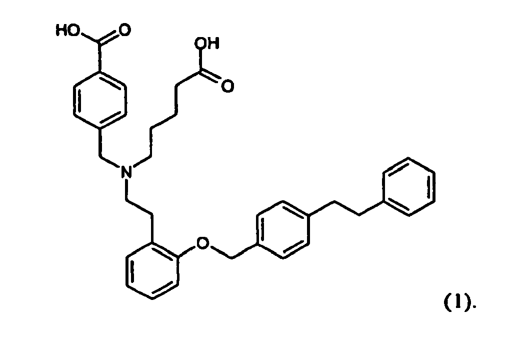

Verbindung 1 und dessen Wirkung auf die lösliche Guanylatcyclase wurden erstmals in WO 01/19780 beschrieben. Die WO 01/19780 beschreibt jedoch keine pharmazeutische Zubereitungen, die zur parenteralen Verabreichung geeignet sind. Insbesondere zur Behandlung von Patienten auf Intensivstationen, die zur oralen Aufnahme nicht befähigt sind, bedarf es einer solchen parenteral verabreichbaren Infusionslösung.

Für eine parenterale Anwendung sprechen insbesondere zwei Gründe:
1) Eine orale Anwendung der Substanz ist aufgrund der kurzen Halbwertzeit der Substanz nur bei mehrmals täglicher Einnahme möglich. Gleichzeitig steht der Entwicklung einer klassischen Retardformulierung entgegen, dass die Substanz nur aus dem oberen Teil des Gastrointestinaltraktes in ausreichenden Mengen resorbiert wird.
2) Verbindung I wird für die Anwendung bei akuter Herzinsuffizienz entwickelt. Die Applikation des Wirkstoffes erfolgt hier üblicherweise in der Intensivmedizin. Bei entsprechenden Intensivpatienten ist eine orale Arzneistoffapplikation schwierig. Die Verabreichung einer i.v. Infusion hingegen stellt die Compliance sicher und ermöglicht eine individuell angepasste exakte Dosierung. Daher ist eine i.v. Lösung in diesem Fall die Formulierung der Wahl.

Im Rahmen der Entwicklungsarbeiten wurde gefunden, dass die Löslichkeit in üblichen physiologischen Lösungsmitteln nicht ausreichend ist, um physikalisch stabile Lösungen zu erhalten.

Aufgabe ist es, trotz dieses Problems verträgliche und gleichzeitig lagerstabile Infusionslösungen zu formulieren. Die Verwendung von Hilfsstoffen ist dabei auf Substanzen beschränkt, die für die weltweite Zulassung eines parenteralen Arzneimittels geeignet sind.

Es wurde gefunden, dass eine pH-Einstellung mit üblichen Puffern nicht zu einer stabilen Formulierung mit ausreichender Löslichkeit führt.

Überraschenderweise bildet Verbindung 1 mit Amino-2(hydroxymethyl)-2-propandiol-1,3 (Trometamol) ein Salz, das eine ausreichende Löslichkeit bei einem pH Wert unterhalb von 9,0 aufweist und somit als Basis für eine stabile und physiologisch verträgliche Formulierung dienen kann. Trometanol ist dem Fachmann bekannt. Gleichzeitig hat die Verwendung von Trometamol den Vorteil, dass ein Absinken des pH Wertes während der Lagerung, und eine damit verbundene Präzipitation des Wirkstoffes aufgrund der herabgesetzten Löslichkeit, durch die gute Pufferwirkung von Trometamol im pH Bereich zwischen 8 - 9 verhindert wird.

Die sinnvolle tägliche Dosis für Verbindung 1 liegt im Bereich 2 bis 20 mg, in einer 50 ml Flasche. Aus diesen für den Wirkstoff sinnvollen Dosisvorgaben (2 - 20 mg in 50 ml) ergibt sich eine Zielwirkstoffkonzentration in der Lösung im Bereich zwischen 0,04 und 0,4 mg/ml. Die Löslichkeit des Wirkstoffs in einem geeigneten Vehikel bei Raumtemperatur sollte mindestens um den Faktor 3 höher liegen (Sicherheitszuschlag), insbesondere um auch bei einer Kühllagerung der Lösungen Präzipitationen aufgrund einer zu geringen Löslichkeit zu vermeiden.

Aufgrund mehrerer ionisierbarer Gruppen der Verbindung 1 (pKₛ₁: 4,0 / pKₛ₂: 4,7 / pKₛ₃: 8,3), wurden Versuche durchgeführt, die durch Einstellen des pH Wertes auf Werte größer als pH 7,4 die Löslichkeit verbessern. Der Erhöhung des pH Wertes sind jedoch durch die Anwendung als Infusion Grenzen gesetzt. pH Werte ab pH 9.0 sind in Bezug auf die physiologische Verträglichkeit grenzwertig. Der Einsatz üblicher Puffer und Basen führte nicht zu einer gangbaren Formulierung. Tabelle 1 zeigt die Löslichkeiten von Verbindung 1 in verschiedenen Puffermedien.

**Tabelle 1: Löslichkeit von Verbindung I in verschiedenen Puffermedien**

| Name des Puffers | Ist-pH-Wert | Sättigungslöslichkeit [mg/ml] |
|---|---|---|
| Phosphat-Puffer-Lösung | 6,0 | 0,0002 |
| Tetrabutylammonium-Puffer-Lösung | 7,0 | 0,0128 |
| Phosphat-Puffer-Lösung | 7,2 | 0,0084 |
| Phosphat-Puffer-Lösung | 7,4 | 0,0215 |
| Saline-Phosphat-Puffer | 7,4 | 0,0190 |
| Citronensäure-Phosphat-Puffer | 7,4 | 0,0131 |
| Phosphat-Puffer-Lösung (0,2 M) | 7,5 | 0,0158 |
| Phosphat-Puffer-Lösung (0,3 M) | 7,5 | 0,0013 |
| HEPES-Puffer-Lösung | 7,5 | 0,0358 |
| Natrium-Citrat-Puffer-Lösung | 7,8 | 0,1601 |
| Phosphat-Puffer-Lösung | 8,0 | 0,0032 |
| Citronensäure-Phosphat-Puffer | 8,0 | 0,0642 |
| Phosphat-Puffer-Lösung | 9,0 | 0,6826 |
| Ammonium-Chlorid-Puffer-Lösung | 9,5 | 75,7 |
| Diethanolamin-Puffer-Lösung | 10,0 | 89,8 |

Trometamol kann in der Formulierung grundsätzlich in einer Konzentration von 0,002 bis 0,2 M eingesetzt werden. Als besonders günstig hat sich eine Konzentration von 0,01 M erwiesen. Bei zu niedrigen Konzentrationen ist die Pufferwirkung nicht ausreichend, um eine stabile Formulierung zu gewährleisten. Bei zu hohen Konzentrationen ist verstärkt mit unerwünschten pharmakologischen Wirkungen zu rechnen, da die Pufferbelastung auf den physiologischen Blutpuffer zu hoch ist. In höheren Konzentrationen wird Trometamol intravenös als Wirksubstanz gegen Hyperacidität eingesetzt. Dabei wird vom Hersteller (Braun, Melsungen) empfohlen eine Konzentration von 0.3 M in Infusionen nicht zu überschreiten. Aus pharmakologisch-toxikologischer Sicht ist somit eine möglichst niedrige Trometamol Konzentration anzustreben.

Eine Konzentration der Verbindung 1 von 0,5 mg/ml entspricht einer Molarität von 0,001 M, so dass eine vollständige Salzbildung für Konzentrationen bis zu 0,5 mg/ml bereits durch Trometamolkonzentrationen von 0,0015 M und darüber gewährleistet ist.

Während bei Konzentrationen im Bereich von etwa 0,2 bis 0,5 mg/ml der Einsatz von Trometamol in den Lösungen essentiell ist für die Löslichkeit von Verbindung 1, ist auch bei niedriger konzentrierten Lösungen von Verbindung 1 der Einsatz von Trometamol als Hilfsstoff für Infusionslösungen von Vorteil.

Verdünnungsversuche an Lösungen mit einem Wirkstoffgehalt von 0,05 mg/ml zeigten, dass diese nur dann über einen weiten Bereich pH-stabil und somit problemlos verdünnbar sind, wenn sie mit einer ausreichenden Menge an Trometamol gepuffert werden.

Ohne Verwendung von Trometamol und bei Einsatz von Trometamol in einer Konzentration von nur 0,001 M fällt der pH Wert schon bei geringer Verdünnung (1+5) stark ab. Hier besteht ein hohes Risiko, dass der Wirkstoff beim Verdünnen mit anderen Infusionslösungen ausgefällt wird (Tabelle 2 a-c).

**Tabelle 2: Verdünnbarkeit von Lösungen enthaltend Verbindung 1**

| a) Ohne Trometamol: | | | |
|---|---|---|---|
| | | | |

| **Mischungsverhältnis** | **1+5** | **1+10** | **1+250** |
|---|---|---|---|
| Verdünnungsmedium | NaCl-Lsg | NaCl-Lsg | NaCl-Lsg |
| Endvolumen nach Verdünnung | 251 ml | 275 ml | 300 ml |
| pH-Wert vor Verdünnen | 8,51 | 8,51 | 8,51 |
| pH-Wert des Verdünnungsmediums | 6,18 | 6,21 | 6,14 |
| pH-Wert nach Verdünnen | 6,40 | 6,58 | 6,70 |
| | | | |

| b) Mit Trometamol 0,001 molar: | | | |
|---|---|---|---|
| | | | |

| **Mischungsverhältnis** | **1+5** | **1+10** | **1+250** |
|---|---|---|---|
| Verdünnungsmedium | NaCl-Lsg | NaCl-Lsg | NaCl-Lsg |
| Endvolumen nach Verdünnung | 251 ml | 275 ml | 300 ml |
| pH-Wert vor Verdünnen | 8,69 | 8,69 | 8,69 |
| pH-Wert des Verdünnungsmediums | 6,25 | 6,06 | 6,12 |
| pH-Wert nach Verdünnen | 8,43 | 8,05 | 6,61 |

| c) Mit Trometamol 0,01 molar: | | | |
|---|---|---|---|
| | | | |

| **Mischungsverhältnis** | **1+5** | **1+10** | **1+150** |
|---|---|---|---|
| Verdünnungsmedium | NaCl-Lsg | NaCl-Lsg | NaCl-Lsg |
| Endvolumen nach Verdünnung | 251 ml | 275 ml | 300 ml |
| pH-Wert vor Verdünnen | 8,74 | 8,74 | 8,74 |
| pH-Wert des Verdünnungsmediums | 6,06 | 6,01 | 5,95 |
| Aussehen der Lösung | klar | klar | klar |

In Titrationsversuchen konnte gezeigt werden, dass eine Säurebelastung mit 0,01 N Salzsäure bei Lösungen ohne Trometamol bereits bei Zugabe von weniger als 1 ml zum gesamten Inhalt einer 50 ml Infusionsflasche zu einem so starken pH Abfall führt, dass der Wirkstoff ausfällt. Bei einem Pufferzusatz von 0,001 M Trometamol sind Ausfällungen bei Zugabe von ca. 5 ml 0,01 N Salzsäure zu beobachten. Die Lösung mit einem Trometamolgehalt von 0,01 M hingegen ist bis zu einer Säurezugabe von mehr als 35 ml 0,01 N Salzsäure stabil gegen Präzipitationen.

Lösungen, die Verbindung 1 in einer Konzentration von 0,05 mg/ml und Trometamol in einer Konzentration von 0,01 M enthalten, können problemlos über einen weiten Bereich mit Standardinfusionslösungen verdünnt werden. Dazu wurden die in Tabelle 3 dargestellten Verdünnungsversuche durchgeführt:

**Tabelle 3: Verdünnbarkeit von Lösungen enthaltend Verbindung 1**

| a) Verdünnungen mit Kochsalzlösung: | | | | | | |
|---|---|---|---|---|---|---|
| **Mischungsverhältnis** | **1:2** | **1:4** | **1:10** | **1:20** | **1:40** | **1:200** |
| Verdünnungsmedium | NaCl-Lsg | NaCl-Lsg | NaCl-Lsg | NaCl-Lsg | NaCl-Lsg | NaCl-Lsg |
| Endvolumen nach Verdünnung | 10 ml | 20 ml | 50 ml | 100 ml | 200 ml | 1000 ml |
| pH-Wert nach Verdünnen | 8,67 | 8,65 | 8,57 | 8,54 | 8,40 | 7,85 |

| b) Verdünnungen mit anderen Medien: | | | | | | |
|---|---|---|---|---|---|---|
| **Mischungsverhältnis** | **1:10** | **1:10** | **1:10** | | | |
| Verdünnungsmedium | GlucoseLsg | Wasser | Placebo Lsg | | | |
| Endvolumen nach Verdünnung | 50 ml | 50 ml | 50 ml | | | |
| pH-Wert nach Verdünnen | 8,37 | 8,61 | 8,72 | | | |

Alle Verdünnungsversuche wurden doppelt durchgeführt. Bei keiner der untersuchten Lösungen traten Ausfällungen auf. Es zeigte sich, dass ein deutlicher Abfall der pH-Werte auf den Bereich unterhalb von 8,0 erst bei Verdünnungen von stärker als 1:40 auftreten. Bei derart starken Verdünnungen ist ein Ausfallen des Wirkstoffs nicht mehr zu befürchten, da durch die große Menge an Verdünnungsmedium die Löslichkeitsgrenze von Verbindung 1 nicht mehr erreicht wird.

Es ist durch die Erfindung möglich, stabile und verträgliche Infusionslösungen enthaltend Verbindung 1 herzustellen. Die Infusionslösungen können entweder unverdünnt appliziert werden oder verdünnt z.B. als Bypass-Infusion mit anderen Standardinfusionslösungen wie isotonischer Kochsalz- oder Glucoselösung.

Vorteil der verdünnten Applikation ist, dass ein größerer Spielraum für Konzentrations- und Dosierungsanpassungen für eine optimale, individuell auf den Patienten zugeschnittene Arzneimitteltherapie besteht.

Gegenstand der Erfindung ist somit eine wässrige Arzneimittelformulierung die 4-[((4-Carboxybutyl)-{2-[(4-phenethylbenzyl)oxy]phenethyl}-amino)methyl]benzoesäure (Verbindung 1) oder ein Salz davon und Amino-2(hydroxymethyl)-2-propandiol-1,3 (Trometamol) enthält.

Physiologisch unbedenkliche Salze können Metall- oder Ammoniumsalze der Verbindung (1) sein. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Eine wässrige Arzneimittelformulierung im Sinne der vorliegenden Erfindung ist eine Formulierung, die im wesentlichen Wasser als Lösungsmittel enthält. Sie kann jedoch je nach zu verabreichendem Infusionsvolumen gegebenenfalls mit Wasser mischbare organische Lösungsmittel in einem Anteil von bis zu 50 % (M/V) bevorzugt weniger als 30 % (M/V) enthalten, sofern sie nicht zu einer Beeinträchtigung der physiologischen Verträglichkeit der Formulierung führen. Besonders bevorzugt enthält die wässrige Arzneimittelformulierung der Erfindung im wesentlichen keine organischen Lösungsmittel.

Die wässrige Arzneimittelformulierung der Erfindung enthält zweckmäßig 0,0005 % (M/V) (0,0001 % M/V meint 0,001 g/100ml) bis 1% (M/V), bevorzugt 0,0025 bis 0,25 % (M/V), besonders bevorzugt 0.005 bis 0.025 % (M/V) der Verbindung (1) oder deren Salze. Diese Gewichtsmengen sind auf das Gesamtvolumen der Formulierung bezogen.

Die Menge des erfindungsgemäß verwendeten Isotonisierungsmittels wird zweckmäßig so ausgewählt, dass Zubereitungen mit einer Tonizität bis zu 430 mOsmol/kg, bevorzugt 250 bis 330 mOsmol/kg erhalten werden.

Die wässrige Arzneimittelformulierung der Erfindung dient zweckmäßig zur parenteralen Applikation. Die parenterale Applikation schließt z.B. die intravenöse, intraarterielle, subkutane, intramuskuläre sowie die intraperitoneale Verabreichung ein, wobei der intravenösen Verabreichung die größte Bedeutung zukommt. Als Dosis werden zweckmäßig 24µg bis 24 mg Wirkstoff für eine 1x tägliche intravenöse Infusion erachtet. Das täglich verabreichte Infusionsvolumen sollte bis 200 ml nicht übersteigen.

Die wässrige Arzneimittelformulierung der Erfindung kann zusätzlich zu den erfindungsgemäß verwendeten Inhaltsstoffen weitere auf dem Gebiet der parenteralen Applikationsformen übliche Hilfsstoffe, wie z.B. Säuren und Basen zur Einstellung des pH-Wertes sowie übliche Konservierungsmittel, Löslichkeitsverbesserer und Antioxidantien enthalten.

Es ist durch die Erfindung möglich, stabile und verträgliche Infusionslösungen mit dem Wirkstoff der Verbindung (1) herzustellen. Es können einfach zu handhabende, fertige Infusionslösungen formuliert werden. Die Bereitstellung der Lösungen kann sowohl in Form von Glas-Infusionsflaschen oder Ampullen als auch in Form flexibler Infusionsbeutel oder geblasener bottelpack^{®} Verpackungen etc. erfolgen.

### Ausführungsbeispiele

### Beispiel 1: Infusionslösung 0,5 mg/ml

Ein 50 1 Bulkansatz enthält:

| Zusammensetzung | Funktion | Masse (g) |
|---|---|---|
| **Wirkstoff** | | |
| Verbindung 1 | Wirkstoff | 27,0000 |

| **Hilfstoffe** | | |
|---|---|---|
| Salzsäure 1 M* | pH Einstellung | 111,2016 |
| | | |
| Natiumchlorid | Isotonisierung | 486,0000 |
| | | |
| Natronlauge 0.1 M* | pH Einstellung | 675,0000 |
| | | |
| Trometamol | Löslichkeitsverbesserung, pH Stabilisierung | 65,4156 |
| | | |
| Wasser für Injektionszwecke | Lösemittel | 52889,18 |
| **Gesamtfüllmenge** | | **54253,80** |

| | | |
|---|---|---|
| **(entspricht 50000 ml)** | | |

Der Wirkstoff wird in einem Behälter aus Glas oder Stahl in der angegebenen Menge Natronlauge gelöst.

Dann werden Trometamol, Salzsäure und 5 % der Gesamtwassermenge zusammengegeben und gerührt, bis das Trometamol gelöst ist. Der Soll-pH Wert dieser Lösung liegt zwischen 8,6 und 8,8.

Parallel dazu werden 90 % der Wassermenge vorgelegt und das Natriumchlorid darin gelöst. Mit Natronlauge wird die Lösung auf pH 8,0 - 8,5 eingestellt. Die dafür benötigte Menge an 0,1 N NaOH wird dokumentiert.

Unter Rühren wird schließlich zuerst die Trometamollösung und anschließend das Wirkstoffkonzentrat zugegeben. Der pH Wert der Lösung wird kontrolliert und mit 1 M Salzsäure oder 0.1 Natronlauge auf pH 8,8 eingestellt. Es ist so lange zu rühren, bis eine klare Lösung resultiert.

Die fertige Lösung wird durch ein Filter der Porenweite 0,22 µm (Pall Ultipor Nylon 66) unter 1.6 bar Stickstoff-Überdruck sterilfiltriert, in Glasinfusionsflaschen a 50 ml abgefüllt und mittels PTFE-kaschierter Chlorbutylkautschuk-Infusionsstopfen und Bördelkappen aus Aluminium verschlossen. Die abgefüllten Lösungen enthaltend Verbindung (1) werden bei 121°C mindestens 20 Minuten autoklaviert.

### Beispiel 2: Infusionslösung 0,05 mg/ml

Eine 50 ml Infusionsflasche enthält:

| Zusammensetzung | Funktion | Masse (g) |
|---|---|---|
| **Wirkstoff** | | |
| Verbindung (1) | Wirkstoff | 0.0025 |

| **Hilfstoffe** | | |
|---|---|---|
| Salzsäure 1 M | pH Einstellung | 0.103 - 0.150 |
| | | |
| Natiumchlorid | Isotonisierung | 0.450 |
| | | |
| Natronlauge 0.1 M | pH Einstellung | 0.063 - 0.150 |
| | | |
| Trometamol | Löslichkeitsverbesserung, pH Stabilisierung | 0.061 |
| | | |
| Wasser für Injektionszwecke | Lösemittel | 49.4165 - 49.5505 |
| **Füllmenge** | 50.0 ml | = 50.23 |
| | | |
| Überfüllung | 1.0 ml | |
| (um die Entnahme von 50 ml sicherzustellen) | | |
| **Gesamtfüllmenge** | 51.0 ml | = 51.23 |

Die Herstellung erfolgt analog zu Beispiel 1.

### Beispiel 3: Infusionslösung 0,125 mg/ml

Ein Liter Bulklösung enthält:

| Zusammensetzung | Funktion | Masse (g) |
|---|---|---|
| **Wirkstoff** | | |
| Verbindung (1) | Wirkstoff | 0,1250 |

| **Hilfstoffe** | | |
|---|---|---|
| Salzsäure 1 M* | pH Einstellung | 2,2666 |
| | | |
| Natiumchlorid | Isotonisierung | 9,0000 |
| | | |
| Natronlauge 0.1 M* | pH Einstellung | 3,1250 |
| | | |
| Trometamol | Löslichkeitsverbesserung, pH Stabilisierung | 1,3333 |
| | | |
| Wasser für Injektionszwecke | Lösemittel | 988,8474 |
| **Gesamtfüllmenge** | | **1004,70 g** |

| | | |
|---|---|---|
| **(entspricht 1000 ml)** * zur Einstellung des pH Wertes auf pH 8,8. Menge kann variieren. | | |

Die Herstellung erfolgt analog zu Beispiel 1.

## Patentansprüche

1. Wässrige Arzneimittelformulierung, die 4-[((4-Carboxybutyl)-{2-[(4-phenethylbenzyl)-oxy]phenethyl}amino)methyl]benzoesäure (Verbindung 1) oder ein Salz davon und Amino-2(hydroxymethyl)-2-propandiol-1,3 (Trometamol) enthält.

2. Wässrige Arzneimittelformulierung nach Anspruch 1, **dadurch gekennzeichnet, dass** sie Verbindung (1) oder ein Salz davon in einer Menge von 0,0005 bis 1 % (bezogen auf die Menge der Verbindung 1) enthält.

3. Wässrige Arzneimittelformulierung nach Anspruch 1 oder 2, **dadurch gekennzeichnet, dass** sie Isotonisierungsmittel in einer Menge enthält, so dass Lösungen mit einer Tonizität von 250 bis 430 mOsmol/kg erhalten werden.

4. Wässrige Arzneimittelformulierung nach irgendeinem der Ansprüche 1 bis 3, **dadurch gekennzeichnet, dass** sie von 0,1 bis 300 mmol/l Amino-2(hydroxymethyl)-2-propandiol-1,3 (Trometamol) enthält.

5. Wässrige Arzneimittelformulierung nach irgendeinem der Ansprüche 1 bis 4 zur parenteralen Applikation bei Menschen und Tieren.

6. Verfahren zur Herstellung einer wässrigen Arzneimittelformulierung der Verbindung (1) oder eines Salzes davon, **dadurch gekennzeichnet, daß** man Amino-2(hydroxymethyl)-2-propandiol-1,3 (Trometamol), zur Formulierung verwendet.

7. Verfahren zur Herstellung einer wässrigen Formulierung der Verbindung (1) oder eines Salzes davon gemäß Anspruch 6, worin eine Lösung der Verbindung (1) oder eines Salzes davon mit einer Konzentration der Verbindung (1) oder eines Salzes davon von mehr als 0,0005 % (M/V) bis zur Sättigungskonzentration der Verbindung 1 oder des Salzes davon bei Raumtemperatur mit einer Amino-2(hydroxymethyl)-2-propandiol-1,3 (Trometamol) enthaltenden Infusionsträgerlösung auf eine für die parenterale Verabreichung geeignete Anwendungskonzentration gebracht wird.

8. Verfahren nach Anspruch 7, worin eine Lösung von Verbindung (1) mit einer Konzentration von mehr als 0,0005 % (M/V) bis 1 % (M/V) (bezogen auf die Menge der Verbindung 1) verwendet wird.

9. Verwendung einer wässrigen Lösung von Verbindung (1) und Amino-2(hydroxymethyl)-2-propandiol-1,3 (Trometamol) in Wasser mit einer Konzentration der Verbindung (1) (bezogen auf die Menge der Verbindung 1) von mehr als 0,0005 % (M/V) bis 1 % (M/V) zur Herstellung eines Arzneimittels zur parenteralen Verabreichung.

## Claims

1. Aqueous pharmaceutical formulation which comprises 4-[((4-carboxybutyl)-{2-[(4-phenethylbenzyl)oxy]phenethyl}amino)methyl]benzoic acid (compound 1) or a salt thereof and amino-2(hydroxymethyl)-2-propane-1,3-diol (trometamol).

2. Aqueous pharmaceutical formulation according to Claim 1, **characterized in that** it comprises compound (1) or a salt thereof in an amount of 0.0005 to 1% (based on the amount of compound 1).

3. Aqueous pharmaceutical formulation according to Claim 1 or 2, **characterized in that** it comprises tonicity agents in an amount such that solutions with a tonicity of from 250 to 430 mOsmol/kg are obtained.

4. Aqueous pharmaceutical formulation according to any of Claims 1 to 3, **characterized in that** it comprises from 0.1 to 300 mmol/l amino-2(hydroxymethyl)-2-propane-1,3-diol (trometamol).

5. Aqueous pharmaceutical formulation according to any of Claims 1 to 4 for parenteral administration in humans and animals.

6. Process for preparing an aqueous pharmaceutical formulation of the compound (1) or of a salt thereof, **characterized in that** amino-2(hydroxymethyl)-2-propane-1,3-diol (trometamol) is used for the formulation.

7. Process for preparing an aqueous formulation of the compound (1) or of a salt thereof according to Claim 6, in which a solution of the compound (1) or of a salt thereof having a concentration of the compound (1) or of a salt thereof of more than 0.0005% (M/V) up to the saturation concentration of compound 1 or of the salt thereof at room temperature is brought with an infusion vehicle solution comprising amino-2(hydroxymethyl)-2-propane-1,3-diol (trometamol) to a use concentration suitable for parenteral administration.

8. Process according to Claim 7, in which a solution of compound (1) having a concentration of more than 0.0005% (M/V) up to 1% (M/V) (based on the amount of compound 1) is used.

9. Use of an aqueous solution of compound (1) and amino-2(hydroxymethyl)-2-propane-1,3-diol (trometamol) in water having a concentration of compound (1) (based on the amount of compound 1) of more than 0.0005% (M/V) up to 1% (M/V) to prepare a medicament for parenteral administration.

## Revendications

1. Formulation médicamenteuse aqueuse, qui contient de l'acide 4[((4-carboxybutyl)-{2-[(4-phénéthyl-benzyl)-oxy]phénéthyl}amino)méthyl]benzoïque (composé 1) ou un sel de celui-ci et du amino-2(hydroxyméthyl)-2-propanediol-1,3 (trométamol).

2. Formulation médicamenteuse aqueuse selon la revendication 1, **caractérisée en ce qu'**elle contient le composé (1) ou un sel de celui-ci en une quantité de 0,0005 à 1 % (rapportée à la quantité du composé 1).

3. Formulation médicamenteuse aqueuse selon la revendication 1 ou 2, **caractérisée en ce qu'**elle contient des agents d'isotonisation en une quantité telle que l'on obtienne des solutions présentant une tonicité de 250 à 430 mosmol/kg.

4. Formulation médicamenteuse aqueuse selon l'une quelconque des revendications 1 à 3, **caractérisée en ce qu'**elle contient de 0,1 à 300 mmole/l de amino-2(hydroxyméthyl)-2-propanediol-1,3 (trométamol).

5. Formulation médicamenteuse aqueuse selon l'une quelconque des revendications 1 à 4 destinée à l'application parentérale chez l'homme et chez l'animal.

6. Procédé de production d'une formulation médicamenteuse aqueuse du composé (1) ou d'un sel de celui-ci, **caractérisé en ce que** l'on utilise pour la formulation du amino-2(hydroxyméthyl)-2-propanediol-1,3 (trométamol).

7. Procédé de production d'une formulation aqueuse du composé (1) ou d'un sel de celui-ci selon la revendication 6, dans lequel on porte une solution du composé (1) ou d'un sel de celui-ci avec une concentration du composé (1) ou d'un sel de celui-ci de plus de 0,0005 % (M/V) jusqu'à la concentration de saturation du composé 1 ou du sel de celui-ci à la température ambiante avec une solution de support d'infusion contenant du amino-2(hydroxyméthyl)-2-propanediol-1,3 (trométamol) à une concentration d'application convenant pour l'administration parentérale.

8. Procédé selon la revendication 7, dans lequel on utilise une solution du composé (1) avec une concentration de plus de 0,0005 % (M/V) à 1 % (M/V) (rapportée à la quantité du composé 1).

9. Utilisation d'une solution aqueuse de composé (1) et de amino-2(hydroxyméthyl)-2-propanediol-1,3 (trométamol) dans l'eau avec une concentration du composé (1) (rapportée à la quantité du composé 1) de plus de 0,0005 % (M/V) à 1 % (M/V) pour la production d'un médicament destiné à l'administration parentérale.
